# EUROPEAN PATENT SPECIFICATION

(11) **EP 1 489 191 B1**
(45) Date of publication and mention of the grant of the patent: **23.04.2008**
(21) Application number: 04447126.6
(22) Date of filing: 19.05.2004
(51) Int. Cl.: C12Q 1/68

(54) **Biological signature of manufactured products**
Biologische Signatur von hergestellten Produkten
Signature biologique des produits fabriqués

(30) Priority: 19.05.2003 EP 03447112
(43) Date of publication of application: 22.12.2004
(73) Proprietor: Eppendorf Array Technologies SA, 5000 Namur (BE)
(72) Inventor: Remacle, José, 5020 Malonne (BE)
(74) Representative: Van Malderen, Joëlle

(56) References cited:
- WO-A-91/17265
- DE-A- 10 145 831
- FR-A- 2 775 693
- US-A- 5 763 176
- US-A1- 2002 155 442
- CAI HONG ET AL: "Electrochemical detection of DNA hybridization based on silver-enhanced gold nanoparticle label" ANALYTICA CHIMICA ACTA, vol. 469, no. 2, 3 October 2002 (2002-10-03), pages 165-172, XP002302770 ISSN: 0003-2670
- ANDREW TATON T ET AL: "Scanometric DNA Array Detection with Nanoparticle Probes" SCIENCE, AAAS. LANCASTER, PA, US, vol. 289, 8 August 2000 (2000-08-08), pages 1757-1760, XP002158394 ISSN: 0036-8075
- CAO Y C ET AL: "Nanoparticles with raman spectroscopic fingerprints for DNA and RNA detection" SCIENCE, AMERICAN ASSOCIATION FOR THE ADVANCEMENT OF SCIENCE,, US, vol. 297, 30 August 2002 (2002-08-30), pages 1536-1540, XP002977805 ISSN: 0036-8075
- KRICKA LARRY J: "Nucleic acid detection technologies - Labels, strategies, and formats" CLINICAL CHEMISTRY, AMERICAN ASSOCIATION FOR CLINICAL CHEMISTRY. WINSTON, US, vol. 45, no. 4, April 1999 (1999-04), pages 453-458, XP002172731 ISSN: 0009-9147

## Description

### Field of the invention

The present invention is related to a method for discriminating a non-biological manufactured product preliminary labelled with a specific biological signature from other non-biological manufactured products comprising a different biological signature or no biological signature.

The present invention is also related to said manufactured product comprising said biological labelling.

### State of the art

The labelling and/or identification of a manufactured product has been developed by using the so-called barcode which is a technique for describing character (alphabeticals, numerals, symbols, etc.) by a combination of parallel (vertical) lines, hereafter referred to as bars of different thickness (width) or arranged at different intervals. Each barcode (symbol) will present binary information, which can be optically read by optical reading means such as an optical scanner.

These barcodes are printed on labels and alike which are attached to books, food products and alike articles or directly printed on the surface of articles (surface of wrappings for articles), thereby permitting them to be imputed by input means (reading means), which quickly input binary information at libraries. The two values (0 and 1) of the binary information are distinguished from each other by differences in reflectance of darker and lighter portions of the barcode, darker portions having a lower reflectance (referred to as black bars) and recognised (read) as one (the key 1 of the binary value), while the light portions having a higher reflectance are recognised (read) as the other (i.e. 0). Recently, it has also been decided to integrate colour in a barcode system.

However, this device still represents the drawbacks of being highly visible upon the manufactured product and should be either printed or presented upon a label and put upon the surface of the manufactured product.

The documents DE 101 45 831 and FR 2775 693 discloses methods for the identification of non-biological manufactured products by labelling said products with a specific nucleotide sequence bound to the external surface or incorporated in said product or one of its elements. The document DE 101 45 831 further describes that the natural information storage capacity of these sequences can be used to provide information about the labelled product.

The US patent 5 763 176 discloses fluorescent microbeads coated with nucleic acids for identifying a product or a person marked thereby. The microbeads have an average size from 0.05 to 5*µ*m. The natural information storage capacity of these nucleic acid sequences can be used to provide information about the labelled product.

WO 91 17265 discloses a method of identifying a material by adding a microtrace additive consisting of DNA molecules. The DNA is covalently linked to hydrophobic beads which are lipophilic and form stable dispersions in liquid hydrocarbons. Such beads generally have a size in the range 1 to 5 *µ*m.

### Aims of the invention

The present invention aims to provide a new method for improving labelling of manufactured products.

A particular aim of the present invention is to provide a simple and efficient method for discriminating a manufactured product with a specific biological signature which is not directly recognised by the consumer and does not necessarily requires the incorporation of a labelling strip upon the surface of the product, but is integrated directly upon the surface of any kind of product or inside the elements forming the product and is made visible or detectable by an appropriate treatment of the product.

A further aim of the present invention is to provide inside or upon the surface of a manufactured product a large number of individual biological signatures, which could be present as recorded biological information units. The method also provides a large number of individual signatures.

A last aim of the present invention is to provide a method for the storage of information by using said biological signature upon a manufactured product as recorded biological information units.

### Summary of the invention

The present invention is related to a method for discriminating a non-biological manufactured product preliminary labelled with a specific biological signature from others non-biological manufactured products comprising a different biological signature or no biological signature.

In the method according to the invention, said specific biological signature is a specific nucleotide sequence bound to the external surface of a product or on a solid material incorporated inside the elements of the product and the method allows the discrimination by making detectable (preferably visible) said specific biological signature through a hybridisation of the specific nucleotide sequence with a complementary nucleotide sequence, said hybridisation resulting in the formation of a metallic precipitate (detectable visible signal) in the product or upon the surface of the product, by detecting the presence of said metallic precipitate and by discriminating said manufactured product from other non-biological manufactured products comprising a different biological signature or no biological signature.

In the method according to the invention the metallic precipitate is preferably obtained through reduction of a metal in the presence of an enzyme, preferably it is a silver precipitate. Said metallic precipitate is preferably a chemical reduction of silver in the presence of colloidal gold particles (having a diameter of less than 20 nm) coupled to the complementary sequence.

However, other metallic precipitates could be also formed by other chemical or biochemical reactions, for instance, ferro(-magnetic) metallic precipitates.

Advantageously, the method comprises the steps of detecting said metallic precipitate by reflection, absorption or diffusion of a light beam (laser beam) upon said metallic precipitate, by a variation of a electromagnetic field, by a variation of heat, by a variation of conductance of an electric current or by other chemical or physical detection methods using appropriate detection and quantification means well known by the person skilled in the art.

The method is advantageously used for obtaining a modification of surface characteristics (i.e plastic surface comprising metallic elements) that can be easily detected by said method.

The preferred detection method is chosen by the person skilled in the art according to the type of metallic precipitate obtained, according to the location where said metallic precipitate is obtained according to the type of surface and/or according to the type of elements present inside the product.

According to a preferred embodiment of the present invention, the specific nucleic sequence is bound by a covalent link to the surface of a nucleotide bearing (solid) material (NBM) incorporated or added upon the surface of the manufactured product or incorporated inside the elements (fibbers, metallic or plastic layers...) of the manufactured product.

Such NBM is added during the manufacturing step of the product and is preferably present on the whole external surface of the product or only in special portions of the product or its surface.

The NBM is preferably incorporated in elements used in the polymerisation of the manufactured products or in the packaging of the manufactured product.

Advantageously, the NBM is composed of microbeads, preferably having a size comprised between about 0.001 and about 1000 µm, more preferably between about 0.1 and 10 about µm, upon which one or more nucleotide sequences can be covalently bound.

Said NBM, including the microbeads, is preferably made of the same organic (polymeric matrix) or inorganic (metallic or even magnetic) compounds than the manufactured products to be recognised.

The specific nucleotide sequence bound to the NBM or directly incorporated upon the surface or in the manufactured product to be recognised, presents a sequence of between about 10 and about 1000 bases, preferably between about 20 and about 200 bases length.

Furthermore, the nucleotide sequence is preferably bound upon the manufactured products or upon the NBM through a linker (spacer), preferably through a nonspecific nucleotide sequence of more than 10 bases, preferably more than 50 bases, or more than 100 bases but preferably lower than 1000 bases, 500 bases or 200 bases. Such linker or spacer can be also a polymeric chain of at least 20 atoms, preferably 50 atoms or more preferably 100 atoms but lower than 1000 atoms, 500 atoms or 200 atoms. Preferably, the polymeric chain is a polyethylene glycol (PEG) chain.

The specificity of discrimination of a biological signature is obtained by using a specific and previously determined (synthetic) nucleotide sequence which in appropriate conditions hybridise to a complementary nucleotide sequence resulting in a signal metallic precipitate which is read by appropriate means.

A "non-biological" manufactured product means any consumable solid product or packaging of a consumable solid liquid or gaseous product, which can be made or used in any kind of industry, including agriculture, but which is not made of biological elements comprising nucleotide acids (such as of animals fungi and plants, or parts (or portions such as meat, skin, seeds, flowers, ...) of said animals or plants). However, the manufactured product can be the packaging of said "biological materials".

Furthermore, said "non biological" manufactured product, its surface or its packaging preferably do not comprise or produce denaturing elements (such as enzymes, organic acids,...) which may destroy the biological signature. The manufactured product is also different from known solid supports which are already used for biological detection, such as micro-well plates, biochips, diagnostic kits, etc.

The specific nucleotide sequence incorporated in or present upon the surface of the manufactured products gives, by specific detection (identification and possibly, quantification), the formation of a signal being a metallic precipitate that allows an identification and therefore the traceability of said manufactured products in various processing and transport steps.

According to the invention, said nucleotide sequence is bound to or is present in the product and fixed to the elements or surface of the product through a common link in order to be accessible (by hybridisation) to its complementary nucleotide sequence that results in the detectable signal (metallic precipitate).

One or two of the sequences (specific integrated nucleotide sequences upon or inside the manufactured products or its corresponding nucleotide sequence) is advantageously labelled with a first member of a binding pair such as biotin, which then react with the second member of said specific binding pair (streptavidin) bound to gold micro particles or anti-biotin antibodies labelled with gold nano particles.

Such gold particles are detected as a catalyst for a silver precipitation. Said silver deposit upon the surface of the manufactured product or inside the elements of the manufactured product results in a darkening of the surface, which is easily detected by various means. Said silver deposit is made of particles having scattering or a diffraction power of a light beam. One particularity of silver is its heat and electric properties which are detectable when different from the surface of the manufactured product on which is bound the signature. Of particular interest is the heat exchange or conductivity.of the silver and its electric high conductivity or capacitance or low resistance.

Said metallic precipitate may also result from an enzymatic reaction such as the one obtained with the use of peroxydase or alkaline phosphatase advantageously coupled to streptavidin. The combination of these two systems of detection is proposed in order to obtain firstly, a colorimetric detection and thereafter, a detection through a metallic precipitate.

Advantageously, the preferred specific nucleotide sequence to be recognised by its complementary nucleotide sequence, comprises nucleotides of between about 5 and about 100 bases, more preferably about 15 and about 40 bases.

The biological signature obtained by the method according to the invention has the advantage of being invisible by human eyes or light detection upon the product, but is specifically detected and/or quantified thereafter. If necessary, said metallic or colorimetric precipitate provides advantageously a specific design, including numbers, drawings, barcodes (system of lines with the same or different widths which are read by a barcode reader or other means).

Typical applications of the invention is to avoid infringement and detect stolen objects by providing biological signatures to unique or expensive materials and goods. Particularly indicated for jewellery, cars or parts of the cars, archaeological and art pieces or book notes. The biological signature is particularly useful tool to provide identification of identity cards, passports or trade name materials. It is also easily applied when incorporated into plastic film especially transparent plastic where the detection is particularly easy to obtain. It is also useful to determine the origin of the production or to follow the traceability of the product. Individual signature of any personal manufactured products is achieved by using a sequence of nucleotides being specific of each person; the sequence can be one part of the personal genomic DNA sequence.

In one particular embodiment, the non-biological manufactured product according to the invention contains one or more different or similar nucleotide sequences, which comprise (recorded) biological information units made of the base of said nucleotide sequence(s), which is therefore used for storage of information. Said (recorded) biological information units is present on the same or upon different nucleotide sequence(s), the special disposition of said biological information units being part of an information storage system. Therefore, another aspect of the present invention is related to a method for the storage of information by using the biological information units present upon said "identical or different" nucleotide sequence put upon the surface of a manufactured product or inside said manufactured product.

Nucleotides sequences are continuous chains of nucleotides formed from 4 different bases.

The number of specific sequences is directly dependent of its length being 4ⁿ with n the number of nucleotides of the sequences. A typical 20 bases long sequence gives a number of different combinations so high making it unique in most circumstances.

Another feature of a nucleotide chain is its possible recognition by a complementary sequence chain. Hybridisation of the complementary chain is the result of the recognition between G=C and A=T.

In a particular embodiment, different NBM are used as means (biological information units) for obtaining an information storage. Nucleotide sequences contains part of the information in the sequence of the AGCT nucleotide. The number of combinations is n^{4'}, n being the number of nucleotides in the sequences. When two or more NBMs are present in the surface of a manufactured product and more particularly on the surface of a packaging, the number of combinations of the NBMs on a given surface is x^{y} with x the number of NBM at one position and y the number of NBMs positions on the surface. Since each NBM represent n⁴ combination of nucleotide sequences, the total number of combination for y NBMs will be n^{4y}. Combination numbers will be very high for nucleotide sequence of 10 (n=10) and with 10 NBM (y=10) on the surface, the possible combination will be 10⁴⁰.

For sequence of 15 nucleotides which is typical the sequence for which a specific hybridisation conditions can be worked out and for 10 NBM, the number of possible combinations will be 10⁶⁰ or almost unlimited.

Therefore, said NBMs coated with nucleotide sequences are particularly efficient to store information. One example is a NBM containing a sequence of 15 nucleotides which is a 5 times repeat of 3 nucleotides. 64 of such NBM are constituted and used for information writing and storage. The NBM are then used as 64 bits and organised on the surface as bytes for storage, in the same way as bytes are used on computer or CD-ROM. The 5 times repeat is given as an example, the number of repeats being between 1 and 20, preferably between 3 and 10 and the number of different nucleotides is 2, 3, 4.

In another embodiment the NBM contain a sequence of 16 nucleotides which is a 4 times repeat of 4 nucleotides. 256 of such NBM are constructed and used for information writing and storage. The NBM beads are delivered on the surface of the packaging as individual leads or as row of beads. The reading is done by detection of the beads labelled with specific label probes or after hybridisation with specific label probes or after hybridisation with specific labelled complementary nucleotide sequences.

In one particular application, the NBM contain spaces for increasing availability of reaction with their corresponding specific probes as explained here above. According to the invention, the person skilled in the art may also use nucleotide sequences.as a source of writing, storing and reading information which are now written, stored and read as binary information. The use of 4, 64 or even 256 or even higher number of information units (BIU) makes this storage of information particularly efficient (BIU= Biological Information Units). As explained here above the BIU will store any amount of information either as single NBM or as combination of several NBMs.

In the method of the invention, a large number of possible sequences is used and their specific recognition by complementary sequence is obtained in order to label products especially manufactured products in a very specific and even individual way.

The invention is especially useful for labelling produced product and to be able to identify them even after their incorporation as pieces in another elaborated product.

A nucleotide sequence of at least 5 bases is bound to solid substrate being for example glass or silica beads in order to obtain a nucleotide bearing material (NBM) .

Biotin molecules are attached to the oligonucleotide used for the specific hybridisation with the nucleotide sequence present on the NBM. Biotin is then recognised with streptadin-monogold particles or anti-biotin monogold particles and detected as such if sufficient particles are present on the surface. A precipitation of Ag particles is preferably performed in order to amplify the particles size until about 0,8 mum. size. The detection of these particles is best observed by using a light scattering or diffraction detection.

A typical detector used is a laser beam illuminating the surface of the product and a photodiode located laterally to detect the diffracted light. The detection is obtained with transparent material such as polycarbonate or polyethylene, polyacryate, polymethyl, metacrylate or with opaque or dark material with low diffraction yield.

The inventors have also discovered that the nucleotide sequence present on the NBM is much better detected, when present at the end of a linker (spacer) fixed upon the support (but which should be not too long). The detection of the biological signature is better when the said specific sequence is located at one end of a 5 to 500 nucleotide spacer sequence. In one particular embodiment, a polymer linker being either a monochain or a ramified polymer (like a branched polymer), is being grafted with the specific nucleotide. A long single polynucleotide strand chain is considered as containing a linker if only one part of the sequence is used for the detection with a complementary labelled probe. A typical chain length of between 50 to 500 bases is easily produced by PCR amplification and can be specifically detected with a probe of between 10 to 30 bases. Long linkers or spacers have the drawbacks of being possibly cut by overbreaking during the product manufacturing thus decreasing the detection yield. Breaks are due to shearing forces, overheating and particular wavelength radiations. Example 1 provides an example of NBM being glass beads activated in order to fix capture probes. Similarly, polystyrene beads are activated by oxidation by KMnO₄ and the carboxylated functions will bind the amino-probes in the presence of coupling agents such as carbodiimide.

Rather than being fixed on a solid material like beads, the nucleotides are fixed directly on the polymers chains or on the monomers of said plastic elements and incorporated to as such during the manufacturing of the product or in some of its part. Monomers or polymers with isothiocyanate, acrylate, epoxyde, aldehyde or other reactive groups are substituted with aminated nucleotides (obtained by chemical synthesis or after amplification by PCR by using one amino ended primer). The nucleotide substituted monomers or partially polymerised or polymers are then added in the manufacturing process of one part at least of the manufactured product. Such manufacturing process include moulding or extracting step. In order to optimise the use of NBM, it is preferred to add the NBM (with the nucleotide sequence or not) in the mould or in the extruder in such a way as to obtain the NBM on the surface of the product so as to be detectable there after.

Another preferred method is to use the nucleotide bearing polymer as an external coating of the surface of the manufactured product. Monomers are also usable by being applied on the product surface by process like spin coating and then polymerisation. One preferred process is coating followed by UV irradiation to initiate the polymerisation process. A lot of different monomers, especially having vinyl groups are susceptible to polymerize either as linear or three dimensional molecules after initialisation by UV light.

In one particular application, the deposition of the polymers at the surface of the product is spatially specific making the spatial arrangement of the nucleotide polymer typical of the product as explained here above for the NBM. One of such typical arrangement is lined of various size which after labelling are read by a code bar reader. The invention is not limited to a specific spatial arrangement since other geometrical forms such as squares, dots, rectangles or other can give specific features. A favourite application is the deposit of the NBM beads through an inject or piezo base delivery system automate in order to provide the spatially arrangement of the signal on the surface of a product.

The present invention will be described in detail in the following non limiting examples

### Example 1: Binding of capture nucleotide sequences on silica beads

### Glass activation

Silica beads bearing silanol functions were first grafted with olefinic silane coupling agent in order to cover the surface with olefinic groups. Two grams of silica sample was immersed for 1h in a 3% anhydrous toluene solution of 7-octenyltrichlorosilane (ABCR (Germany, Karlsruhe). The samples were then cleaned three times by dipping in fresh toluene under mild agitation and three times in acetone to remove the excess physisorbed molecules, then dried in oven at 70 °C during 45 min.

### Olefinic oxidation

The olefinic functions present either on glass or polymers were oxidized in the following way. Silica beads were dipped into a solution of 0.01M Phosphate buffer at pH 7.4 containing 0.5 M NaI04 and 20 mM KMnO4 under mild agitation during 1h, washed six times with water, three times with acetone and dried at 20°C. Sample was stored at 4°C.

### Grafting of aminated DNA nucleotide sequences on silica beads

### Capture nucleotide sequence synthesis

Capture nucleotide sequences are synthesized by PCR using primers and method described by Zammatteo et al. (1997). Anal. Biochem. 253, 180-189). One primer Mie4 bears an amine group at its 5' terminus and the length of amplicons is 255 bp. The sequences of the primers were MiE4: ccaagcggcctctgataaccaag and MiE6: gtacaggggactctgggggtgac....

The amplified sequence was a part of the major immediate early gene of cytomegalovirus genome (HCMV strain AD169). During the PCR process, biotinylated nucleotides are incorporated to generate 255 base long biotinylated amplicons bearing an aminated end. Amplified DNA is separated from unincorporated nucleotides and primers by chromatography on High Pure PCR Product Purification Kit. DNA concentration is then estimated on agarose gel electrophoresis.

### Binding of single stranded capture nucleotide sequences to aldehyde sample

500 mgr of silica beads are diluted to a concentration of 100 nM in SSC 3X buffer pH 5, 0.05% SDS. Grafting is performed during 1 h under mild agitation. After incubation, sample is washed once with 0.2% SDS, twice with water, then incubated for 5 min with sodium borohydride solution [2.5 mg/ml dissolved in a PBS/Ethanol solution (75/25)], once with water and finally 3 min in boiling water to obtain single stranded nucleotide sequences on the surface. Beads are resuspended in 50 ml of water.

### Test of DNA binding on beads.

To confirm the presence of biotinylated DNA on the surface of beads (possibly integrated in the packaging of a manufactured product such as a cigarette box), the detection of biotin on beads with DNA bound and on negative control beads without DNA. 2µl of silice beads are washed 4X2min with a 10 mM maleate buffer containing 15mM NaCl and 0.1% tween pH 7.5 (Washing buffer). The beads are then incubated for 45 min with streptavidin-HRP conjugate diluted 1000X in a blocking solution. The beads are then washed 5X2min in the same washing buffer then incubated 10 min in TMB solution to detect a blue colour appearing were HRP is present. The solution becomes yellow and 100µl of this solution is quantified by a colorimeter at 450 nm. An optical density of 3.5 for beads with DNA bound and an optical density of 0.2 for negative control beads can be obtained upon the surface of the packaging.

### Example 2: Identification of the presence of a DNA sequence on the beads

Detection for the presence of the particular DNA sequence on the beads is performed in the same way as in example 1 but with the beads bearing non-biotinylated DNA strand. The detection is then performed with DNA binding performed using the complementary sequence labelled with biotin as described by Zammatteo et al. (1997) ( Anal. Biochem. 253, 180-189).

For the detection of the DNA sequence on a surface of a manufactured product, the DNA-bearing beads were deposit inside a polypropylene sheet and on a cellulose surface. The polymers surfaces are sticked to the surface of the manufactured product or its packaging.

The presence of the DNA on the surface of the beads is detected in colorimetry following this protocol: Slides are washed 4X2min with a 10 mM maleate buffer containing 15mM NaCl pH 7.5 (Washing buffer). The slides are then incubated for 45 min with streptavidin-gold nanoparticle conjugate diluted 100X in a 100mM maleate buffer containing 150mM NaCl and 0.01% milk powder. The slides are then washed 5X2min in the same washing buffer.

The slides are incubated at room temperature for 10 min in 800*µ*l of Silver Blue solution in the combination of 1/1 volume of Silver Blue A and Silver Blue B (EAT, Namur, Belgium). The silver blue allows the precipitation of silver catalyzed by the presence of the gold nanoparticles present on the DNA to be detected

The reaction of Silver precipitation is stopped by washing the slides in water for 2 min.

## Claims

1. A method for discriminating a non-biological manufactured product preliminary labelled with a specific biological signature from other non-biological manufactured products comprising a different biological signature or no biological signature,
said specific biological signature being a specific nucleotide sequence bound to the external surface of a product or on a solid material incorporated inside the solid elements of the products,
- by making detectable said specific biological signature through a hybridisation of said specific nucleotide sequence with a complementary nucleotide sequence, said hybridisation resulting in the formation of a metallic precipitate in the product or upon the surface of the product,
- by detecting the presence of said metallic precipitate and
- by discriminating said manufactured products from other non-biological manufactured products comprising a different biological signature or no biological signature.

2. The method of claim 1, wherein the metallic precipitate is obtained through a reduction of a metal in the presence of an enzyme.

3. The method according to the claim 1 or 2, wherein the detectable metallic precipitate is a chemical reduction of silver in the presence of colloidal gold particles coupled to the complementary sequence.

4. The method according to any of the preceding claims 1 to 3, wherein the metallic precipitate is detected by a reflection, an absorption or a diffusion of a light beam upon said precipitate, a variation of an electro-magnetic field, a variation of heat or a variation of conductance of an electric current.

5. The method according to anyone of the preceding claims, wherein the specific nucleotide sequence is bound by a covalent link to a surface of a nucleotide bearing (solid) material (NBM) incorporated and added upon the surface of the manufactured product or incorporated inside the elements of the manufactured product or incorporated inside the elements of the manufactured product.

6. The method according to claim 5, wherein the nucleotide bearing (solid) material (NBM) is made of microbeads having a size comprised between 0.01 and 1000 µm, more preferably between 0.1 and 10 µm.

7. The method according to any of the preceding claims , wherein the specific nucleotide sequence is attached to the surface of the product, of or to the solid material through a linker of more than 10 bases, preferably more than 50 bases, more specifically more than 100 bases.

8. The method according to any of the preceding claims, wherein the nucleotide sequence presents a length comprised between 10 and 1000 bases, more preferably between 20 and 200 bases.

9. The method according to any of the preceding claims which further comprises the steps of reading storage information within the non biological manufactured product, said storage information being incorporated into the specific nucleotide sequence of the biological signature.

10. The method according to the claim 9, wherein the Storage Information are present, according to a spatial disposition of the nucleotide bearing material (NBM) upon the product surface.

## Patentansprüche

1. Verfahren zum Unterscheiden eines nicht-biologisch hergestellten Produktes, das vorher mit einer spezifischen biologischen Signatur markiert wurde, von anderen nicht-biologisch hergestellten Produkten, die eine unterschiedliche biologische Signatur oder keine biologische Signatur umfassen,
wobei die spezifische biologische Signatur eine spezifische Nukleotidsequenz ist, gebunden an die Oberfläche von einem Produkt oder an ein festes Material, das im Inneren der festen Elemente von den Produkten eingearbeitet ist,
- über das Erkennbarmachen der spezifischen biologischen Signatur durch eine Hybridisierung der spezifischen Nukleotidsequenz mit einer komplementären Nukleotidsequenz, wobei die Hybridisierung in der Bildung eines metallischen Niederschlages in dem Produkt oder auf der Oberfläche von dem Produkt resultiert,
- über die Erkennung der Anwesenheit des metallischen Niederschlages und
- durch Unterscheiden der hergestellten Produkte von anderen nicht-biologisch hergestellten Produkten, die eine unterschiedliche biologische Signatur oder keine biologische Signatur umfassen.

2. Verfahren nach Anspruch 1, wobei der metallische Niederschlag durch eine Reduktion von einem Metall in der Gegenwart von einem Enzym erhalten wird.

3. Verfahren nach Anspruch 1 oder 2, wobei der erkennbare metallische Niederschlag ein chemisches Reduktionsprodukt aus Silber in der Gegenwart von kolloidalen Goldpartikeln ist, welche an die komplementäre Sequenz gekoppelt sind.

4. Verfahren nach einem beliebigen der vorhergehenden Ansprüche 1 bis 3, wobei der metallische Niederschlag durch Reflexion, Absorption oder Diffusion von einem Lichtstrahl auf den Niederschlag, einer Veränderung von einem elektromagnetischen Feld, einer Veränderung der Wärme oder einer Veränderung der Leitfähigkeit eines elektrischen Stroms erkannt wird.

5. Verfahren nach einem beliebigen der vorhergehenden Ansprüche, wobei die spezifische Nukleotidsequenz durch eine kovalente Bindung gebunden ist an eine Oberfläche von einem Nukleotid-beladenen (festen) Material (NBM), das eingearbeitet und auf die Oberfläche von dem hergestellten Produkt hinzugefügt oder innerhalb der Elemente von dem hergestellten Produkt eingearbeitet ist.

6. Verfahren nach Anspruch 5, wobei das Nukleotid-beladene (feste) Material (NBM) aus Mikrokugeln hergestellt ist, welche eine Größe aufweisen, die zwischen 0,01 und 1.000 µm, noch bevorzugter zwischen 0,1 und 10 µm liegt.

7. Verfahren nach einem beliebigen der vorhergehenden Ansprüche, wobei die spezifische Nukleotidsequenz an der Oberfläche von dem Produkt, auf oder an dem festen Material, durch ein Verbindungsteil aus mehr als 10 Basen, vorzugsweise mehr als 50 Basen, insbesondere mehr als 100 Basen angebracht ist.

8. Verfahren nach einem beliebigen der vorhergehenden Ansprüche, wobei die Nukleotidsequenz eine Länge aufweist, die zwischen 10 und 1.000 Basen, vorzugsweise zwischen 20 und 200 Basen liegt.

9. Verfahren nach einem beliebigen der vorhergehenden Ansprüche, welches ferner die Schritte des Lesens der Speicherinformation innerhalb des nicht-biologisch hergestellten Produktes umfasst, wobei die Speicherinformation in der spezifischen Nukleotidsequenz von der biologischen Signatur eingearbeitet ist.

10. Verfahren nach Anspruch 9, wobei die Speicherinformationen vorhanden sind, gemäß einer räumlichen Anordnung von dem Nukleotid-beladenen Material (NBM) auf der Produkt-Oberfläche.

## Revendications

1. Procédé pour différencier un produit fabriqué non biologique marqué au préalable avec une signature biologique spécifique d'autres produits fabriqués non biologiques comprenant une signature biologique différente ou aucune signature biologique,
ladite signature biologique spécifique étant une séquence de nucléotides spécifique liée à la surface externe d'un produit ou sur un matériau solide incorporé à l'intérieur des éléments solides des produits,
- en rendant détectable ladite signature biologique spécifique par une hybridation de ladite séquence de nucléotides spécifique avec une séquence de nucléotides complémentaire, ladite hybridation aboutissant à la formation d'un précipité métallique dans le produit ou sur la surface du produit,
- en détectant la présence dudit précipité métallique et
- en différenciant lesdits produits fabriqués d'autres produits fabriqués non biologiques comprenant une signature biologique différente ou aucune signature biologique.

2. Procédé selon la revendication 1, dans lequel le précipité métallique est obtenu par réduction d'un métal en présence d'une enzyme.

3. Procédé selon la revendication 1 ou 2, dans lequel le précipité métallique détectable est une réduction chimique d'argent en présence de particules d'or colloïdal couplées à la séquence complémentaire.

4. Procédé selon l'une quelconque des revendications précédentes 1 à 3, dans lequel le précipité métallique est détecté par une réflexion, une absorption ou une diffusion d'un faisceau lumineux sur ledit précipité, une variation d'un champ électromagnétique, une variation de chaleur ou une variation de conductance d'un courant électrique.

5. Procédé selon l'une quelconque des revendications précédentes, dans lequel la séquence de nucléotides spécifique est liée par un lien covalent à une surface d'un matériau (NBM) (solide) portant des nucléotides incorporé et ajouté sur la surface du produit fabriqué ou incorporé à l'intérieur des éléments du produit fabriqué ou incorporé à l'extérieur des éléments du produit fabriqué.

6. Procédé selon la revendication 5, dans lequel le matériau (NBM) (solide) portant des nucléotides est constitué de microbilles ayant une taille comprise entre 0,01 et 1 000 µm, de manière plus avantageuse entre 0,1 et 10 µm.

7. Procédé selon l'une quelconque des revendications précédentes, dans lequel la séquence de nucléotides spécifique est attachée à la surface du produit, du ou au matériau solide par un lieur de plus de 10 bases, de manière plus avantageuse de plus de 50 bases, plus spécifiquement de plus de 100 bases.

8. Procédé selon l'une quelconque des revendications précédentes, dans lequel la séquence de nucléotides présente une longueur comprise entre 10 et 1 000 bases, de manière plus avantageuse entre 20 et 200 bases.

9. Procédé selon l'une quelconque des revendications précédentes qui comprend en outre l'étape consistant à lire des informations de stockage dans le produit fabriqué non biologique, lesdites informations de stockage étant incorporées dans la séquence de nucléotides spécifique de la signature biologique.

10. Procédé selon la revendication 9, dans lequel les informations de stockage sont présentes, selon une disposition spatiale du matériau (NBM) portant des nucléotides sur la surface du produit.
